# EUROPEAN PATENT SPECIFICATION

(11) **EP 3 439 727 B1**
(45) Date of publication and mention of the grant of the patent: **24.03.2021**
(21) Application number: 17713438.4
(22) Date of filing: 23.02.2017
(51) Int. Cl.: A61M 25/01

(54) **STEERING TOOL WITH CONTROLLED DISTAL FLEXIBILITY**
LENKBARES WERKZEUGS MIT EINER KONTROLLERBAREN FLEXIBILITÄT DES DISTALENDES
UN OUTIL DE DIRECTION ORIENTABLE AVEC UNE FLEXIBILITÉ DISTALE CONTROLÉE

(30) Priority: 01.03.2016 US 201615057329
(43) Date of publication of application: 13.02.2019
(73) Proprietor: Bendit Technologies Ltd., 4951024 Petah Tikva (IL)
(72) Inventor: CABIRI, Oz, 4526611 Hod HaSharon (IL); HAMMER, Tal, 5237008 Ramat Gan (IL); MEIRI, Oded, 1920500 Ram-On (IL)
(74) Representative: Jaeger, Michael David
(86) International application number: PCT/IB2017/051040
(87) International publication number: WO 2017/149416

(56) References cited:
- WO-A1-2015/095475
- US-A1- 2011 077 621
- US-A1- 2015 107 396
- US-A1- 2016 015 250

## Description

### FIELD OF THE INVENTION

The present invention generally relates to a steering tool for steering medical devices through body lumens, and particularly to a steering tool with controlled flexibility of the distal portion of the tool.

### BACKGROUND OF THE INVENTION

PCT Patent Application PCT/US2014/071075, to the present inventor, describes a steering tool for steering medical devices through body lumens. The steering tool has an internal tube disposed inside an external tube. The internal and external tubes are arranged for longitudinal axial movement relative to one another. The distal end of the internal tube is fixedly joined to the distal end of the external tube. One or both of the internal and external tubes is slotted near the distal end thereof. The longitudinal axial movement causes bending of the distal ends of the tubes. One or both of the internal and external tubes are slotted near the distal ends thereof. The steering tool provides a distal tip which combines steerability, flexibility and torqueability. The tool eliminates the need for pull/push wires.

The steering tool includes a manipulation handle and a tube manipulator, such as a linear slider. The tube manipulator causes relative axial movement of the internal and external tubes. For example, moving tube manipulator distally causes bending of the distal tip of the internal and external tubes. The tube manipulator may be locked in place and released when desired from the locked position and relocked in place. In the unlocked position, there is free movement during insertion of the steering tool into the body lumen, so that both tubes can move freely and allow the steering tool to bend in accordance with the shape of the body lumen (such as any kind of curved path). After reaching the desired destination in the body lumen, the handle is locked for no more bending. Thus, the steering tool can be used very easily as a guide wire for guiding catheters and other devices.

US-A1-2015107396 describes an actuating member for causing an elongated member for medical use to perform a predetermined curving motion, and a medical instrument including the actuating member. This document may be regarded as s closest prior art and discloses a tool comprising: an internal tube disposed inside an external tube, the internal and external tubes being arranged for longitudinal axial movement relative to one another, wherein a distal end of the internal tube is fixedly joined to a distal end of the external tube; a tube manipulator operative to cause relative axial movement of the internal and external tubes and bending of a distal portion of the internal and external tubes; a locking mechanism operative to switch between a locked position and an unlocked position, wherein in the locked position the internal and external tubes are locked at a particular longitudinal axial position relative to one another and in the unlocked position there is uninhibited free movement of the internal and external tubes relative to one another.

US-A1-2011077621 describes a locking mechanism for a medical device, more particularly a locking mechanism for selectively locking a first elongate member from longitudinal movement relative to a second elongate member of the medical device.

US-A1-2016015250 describes an actuating member for making a medical elongate member perform a predetermined action, and a medical device equipped with the actuating member.

### SUMMARY OF THE INVENTION

The present invention seeks to provide further improvements to the steering tool for steering medical devices through body lumens, as is described more in detail hereinbelow.

There is thus provided in accordance with an embodiment of the present invention a steering tool including an internal tube disposed inside an external tube, the internal and external tubes being arranged for longitudinal axial movement relative to one another, wherein a distal end of the internal tube is fixedly joined to a distal end of the external tube, a tube manipulator operative to cause relative axial movement of the internal and external tubes and bending of a distal portion of at least one of the internal and external tubes, and a locking mechanism operative to switch between a locked position and an unlocked position, wherein in the locked position the internal and external tubes are locked at a particular longitudinal axial position relative to one another, and in the unlocked position there is uninhibited free movement of the internal and external tubes relative to one another, and wherein the locking mechanism includes an adjustment assembly operative to create an intermediate locked position in which there is partially inhibited movement of the internal and external tubes relative to one another, and wherein the adjustment assembly includes a biasing device operative to apply a biasing force on at least one of the internal and external tubes that at least partially inhibits movement of the internal and external tubes relative to one another.

In accordance with an embodiment of the present invention a control knob is operatively connected to the adjustment assembly for switching between the locked, intermediate locked and unlocked positions. The control knob may be lockable at different positions.

In accordance with an embodiment of the present invention the control knob is connected to a stopper which is movable towards an abutment element mounted on at least one of the tubes.

In accordance with an embodiment of the present invention, in the locked position, the biasing device applies a first biasing force against the abutment element sufficient to completely inhibit movement of the internal and external tubes relative to one another, and in the intermediate locked position, the biasing device applies a second biasing force against the abutment element to partially inhibit movement of the internal and external tubes relative to one another.

In the locked position, distal portions of the internal and external tubes are in a fixed deformed orientation, and in the intermediate locked, the distal portions of the internal and external tubes are in a changeable deformed orientation.

### BRIEF DESCRIPTION OF THE DRAWINGS

The present invention will be understood and appreciated more fully from the following detailed description taken in conjunction with the drawings in which:
Fig. 1 is a simplified illustration of a steering tool, in accordance with an
embodiment of the present invention;
Figs. 2A and 2B are simplified illustrations of a handle of the steering tool, in accordance with an embodiment of the present invention, showing a control knob for locking the tubes of the steering tool completely or partially or not at all (i.e., unlocked so the tubes can move freely);
Fig. 2C is an enlarged illustration of the control knob;
Fig. 3A is a simplified cutaway illustration of the steering tool, showing the internal mechanism;
Fig. 3B is a more enlarged cutaway illustration of abutment elements mounted on the inner and outer tubes of the steering tool;
Fig. 3C is a simplified cutaway illustration of the steering tool handle, showing the control knob connected to a stopper which is movable towards the abutment element of one of the tubes for locking the tube;
Figs. 4A and 4B are simplified cutaway illustrations of the steering tool, showing a biasing device mounted between the stopper and the abutment element, wherein the biasing device is in a fully compressed position and the tubes are completely locked;
Figs. 5A and 5B are simplified cutaway illustrations of the steering tool, showing the biasing device in a partially compressed position and the tubes are partially locked;
Figs. 6A and 6B are simplified illustration of a steering tool, in accordance with another embodiment of the present invention;
Fig. 7 is a simplified perspective illustration of a handle of the steering tool, in accordance with another embodiment of the present invention;
Figs. 8A-8C are simplified sectional illustrations of the handle of Fig. 7, in respective undeformed, partially deformed and fully deformed positions for deformation of the tubes of the steering tool;
Fig. 8D is a simplified sectional, perspective illustration corresponding to the fully deformed position of Fig. 8C;
Figs. 9A and 9B are simplified side-view illustrations of a control knob of the handle of Fig. 7, in respective locked and unlocked positions; and
Fig. 10 is a simplified cutaway illustration of a twist knob of the handle of Fig. 7, which is used to control the twisted shape of the tubes of the steering tool.

### DETAILED DESCRIPTION OF EMBODIMENTS

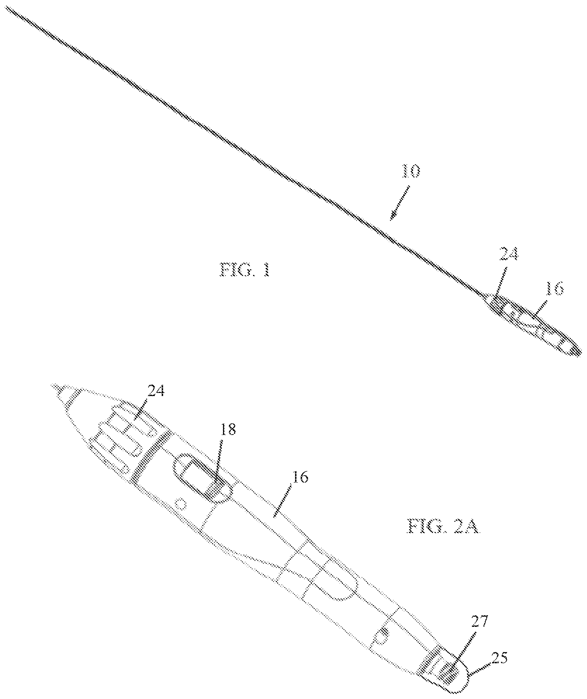

Reference is now made to Figs. 1-2B, which illustrate a steering tool 10, in accordance with an embodiment of the present invention.

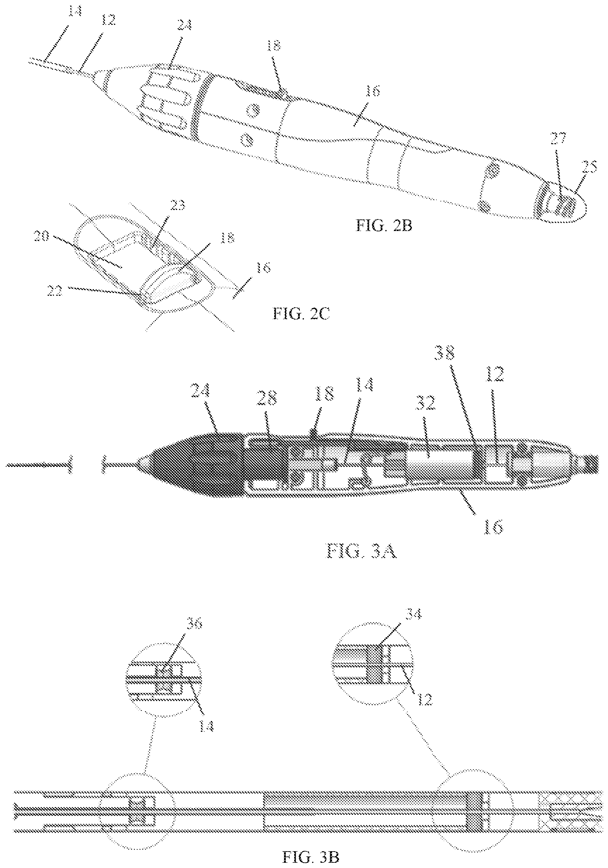

Steering tool 10 includes an internal tube 12 disposed inside an external tube 14 (seen in Fig. 2B). A distal end of internal tube 12 is fixedly joined to a distal end of external tube 14. The term "joined" encompasses any method for attaching the materials of the tubes together, such as but not limited to, welding, ultrasonic welding, thermal bonding, adhesive bonding, molding, and others. The internal and external tubes 12 and 14 are arranged for longitudinal axial movement relative to one another (except for their distal ends which are joined together).

Steering tool 10 includes a handle 16 that has a control knob 18 for locking the tubes 12 and 14 of steering tool 10 completely or partially or not at all (i.e., unlocked so the tubes can move freely), as is described further below. Control knob 18 may be a slidable knob that slides in a channel 20 and which has teeth 22 that can engage ridges 23 formed in the sides of channel 20 (Fig. 2C). In this manner, control knob 18 may be lockable at different positions. In alternative embodiments, control knob 18 may be a rotating element, non-limiting examples of which are given below. The term "knob" encompasses any manipulative element, such us but not limited to, a knob, button, roller, switch and the like. For example, although the embodiment below is described with reference to a mechanical knob, alternatively, the invention may be carried out with an electronic biasing system, in which case the control knob may be an electrical switch, such as a button or touchpad.

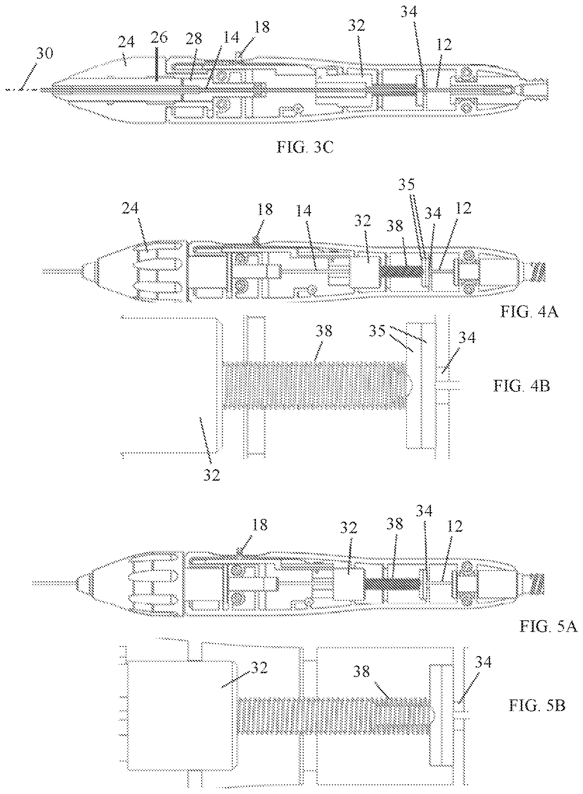

Handle 16 may also include a tube manipulator 24. Rotation of tube manipulator 24 causes longitudinal axial movement of one of the internal and external tubes 12 and 14 relative to one another so as to cause the distal portions of the tubes to bend or curve or otherwise deform, as is now described with reference to Fig. 3C (some parts also indicated in Fig. 3A).

The tube manipulator 24 includes an internal spindle 26 which is in threaded engagement with a tube holder 28 secured to outer tube 14. Rotation of tube manipulator 24 together with internal spindle 26 about a longitudinal axis 30 of the device causes tube holder 28 together with outer tube 14 to move axially along longitudinal axis 30 (the threaded connection converts the rotational movement to axial movement as in the advancement of a screw). The longitudinal axial movement of external tube 14 relative to internal tube 12 causes the distal portions of the tubes to bend or curve or otherwise deform. The tube manipulator 24 could alternatively be connected to the internal tube 12 instead of the outer tube 14 and cause longitudinal axial movement of internal tube 12 relative to external tube 14 to cause the distal portions of the tubes to bend or curve or otherwise deform.

The tube manipulator 24 is used to bend, curve or otherwise deform the distal portions of the tubes, Thus, tube manipulator 24 controls steering and tip location in one plane at a time. Optionally, steering tool 10 may include another manipulator, called a phase shift manipulator 25, which may be a knob in threaded engagement with a threaded shaft 27 connected to internal tube 12. One or both of the internal and outer tubes may be formed with slots. The slots may be phase-shifted with respect to one another (i.e., the slots on one tube may be phase-shifted with respect to one another and/or the slots of one tube may be phase-shifted with respect to the slots of the other tube). Rotation of phase shift manipulator 25 applies torque to internal tube 12, thereby changing the amount of phase shift between the tubes or between the slots, and thereby causing a twisted or spiral shape to the distal tips of the tubes. The operation of tube manipulator 24 (e.g., to cause steering and bending of the distal tips) and the operation of phase shift manipulator 25 (e.g., to cause twisting torque) are independent of each other, thereby providing the surgeon with limitless possibilities of shaping the distal tips to any desired, three-dimensional shape. The twisting torque also changes the stiffness of the bent portion of the tubes.

Steering tool 10 includes a locking mechanism that switches between a locked position and an unlocked position, as will be explained with reference to Fig. 4A (and some of which is illustrated in Fig. 3C). In the locked position, the internal and external tubes 12 and 14 are locked at a particular longitudinal axial position relative to one another, and in the unlocked position there is uninhibited free movement of the internal and external tubes 12 and 14 relative to one another. The locking mechanism includes an adjustment assembly that creates an intermediate locked position in which there is partially inhibited movement of the internal and external tubes 12 and 14 relative to one another.

The locking mechanism includes the control knob 18 which is connected to a stopper 32, and an abutment element 34 mounted on the inner tube 12. As seen in Fig. 3B, another abutment element 36 is mounted on the outer tube 14. Additionally or alternatively, another stopper (or stopper 32) could move towards abutment element 36.

The adjustment assembly includes a biasing device 38 that applies a biasing force on the internal tube 12 and/or external tube 14 that at least partially inhibits movement of internal and external tubes 12 and 14 relative to one another. The biasing device 38 may be, without limitation, a coil spring, leaf spring, or wire or band whose tensile state is changeable (e.g., a nitinol wire or band). In the illustrated embodiment, the biasing device 38 is a coil spring positioned between stopper 32 and abutment element 34 (or more specifically one or more washers 35 placed in front of abutment element 34).

In the locked position, biasing device 38 applies a first biasing force against abutment element 34 sufficient to completely inhibit movement of internal and external tubes 12 and 14 relative to one another. This biasing force is created by moving the control button 18 sufficiently backwards in channel 20 (Fig. 2C). In the intermediate locked position, biasing device 18 applies a second biasing force (weaker than the first biasing force) against abutment element 34 to partially inhibit movement of internal and external tubes 12 and 14 relative to one another. This weaker biasing force is created by moving the control button 18 less backwards in channel 20 (Fig. 2C).

In the case of a rotary control knob, without limitation, the control knob may include a rotatable cam or winding spool that stretches or relaxes the biasing device to adjust the biasing force.

As seen in Figs. 4A and 4B, in the locked position, biasing device 38 is in a fully compressed position and the tubes are completely locked.

As seen in Figs. 5A and 5B, in the intermediate locked position, biasing device 38 is in a partially compressed position and the tubes are partially locked.

In the locked position, distal portions of the internal and external tubes are in a fixed deformed orientation, and in the intermediate locked, the distal portions of the internal and external tubes are in a changeable deformed orientation. In the unlocked position, in which control button 18 is moved to the distal end of channel 20 (Fig. 2C), there is free movement of both tubes. Thus, during insertion of the steering tool 10 into the body lumen, the unlocked position may be used so that both tubes can move freely and allow the steering tool to bend in accordance with the shape of the body lumen (such as any kind of curved path). After reaching the desired destination in the body lumen, the handle is partially or fully locked for no more bending. Thus, the steering tool can be used very easily as a guide wire for guiding catheters and other devices.

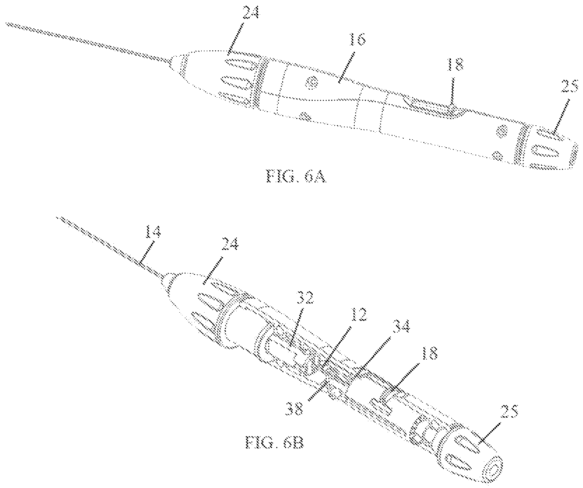

Figs. 6A and 6B illustrate another version of the steering tool described above, with like numerals designating like elements. This embodiment differs from the previous embodiment in the placement of the control knob and type of biasing device used for the adjustment assembly. For example, the biasing device in this embodiment may be a spring wire or a leaf spring as opposed to a coil spring in the previous embodiment.

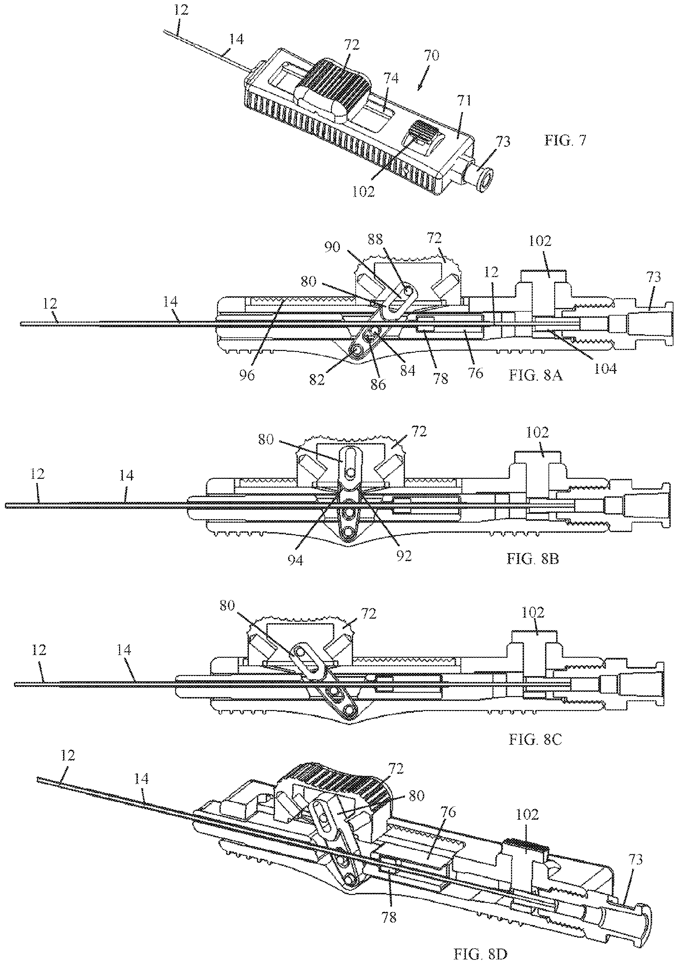

Reference is now made to Figs. 7-8D, which illustrate a handle 70 for deforming the internal and external tubes 12 and 14 of the steering tool described above, in accordance with another embodiment of the present invention. Handle 70 has a housing 71 and may include a medical device connector 73, such as a fluid connector, luer fitting and others, at the proximal end face of housing 71.

Handle 70 includes a control knob 72, which is arranged to slide in a recess 74 formed in the upper face of handle 70. The external tube 14 may be connected to an external tube carrier 76 by means of an adaptor 78. Axial movement of control knob 72 (that is, linear movement along the longitudinal axis of the handle) causes axial movement of external tube 14. Control knob 72 may be linked to external tube carrier 76 by means of an arm 80, which is pivotally connected to housing 71 by a first pivot pin 82 at the bottom of arm 80. Arm 80 is pivotally connected to external tube carrier 76 by a second pivot pin 84, which is arranged to travel in a first channel 86 formed in arm 80. Arm 80 is pivotally connected to control knob 72 by a third pivot pin 88, which is arranged to travel in a second channel 90 formed in arm 80 at the top of arm 80.

Figs. 8A-8C illustrate the travel of handle 70. In Fig. 8A, the handle 70 is at its most proximal position, and in this position, the external tube has not yet been displaced distally, so the internal and external tubes 12 and 14 are undeformed.

In Fig. 8B, the handle 70 has been moved distally to an intermediate position, thereby moving the external tube 14 distally, which partially deforms the internal and external tubes 12 and 14 and makes their distal portions bend partially as described above.

In Fig. 8C, the handle 70 has been moved distally to its most distal position, thereby further moving the external tube 14 distally, which fully deforms the internal and external tubes 12 and 14 and makes their distal portions bend fully as described above.

It is noted that arm 80 may include proximal and distal stoppers 92 and 94 (Fig. 8B), such as chamfered abutment faces formed on proximal and distal portions, respectively, of a sideways protrusion on arm 80. The proximal and distal stoppers 92 and 94 abut against external tube 14 (or alternatively external tube carrier 76) at the end of the proximal and distal travel of control knob 72, respectively.

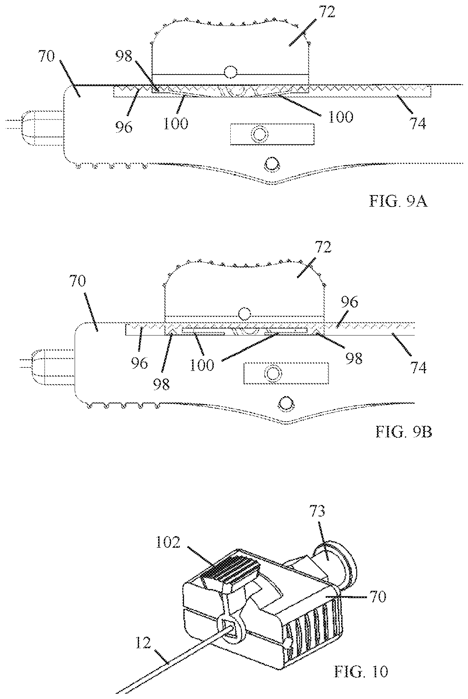

Reference is now made to Figs. 9A and 9B, which illustrate respective locked and unlocked positions of control knob 72. The underside of the upper side rims of recess 74 may be formed with downward-facing teeth 96. Control knob 72 may be include upward-facing teeth 98 formed on two leaf springs 100, each located on one of the sides of control knob 72. As seen in Fig. 9A, when no downward force is exerted on control knob 72, the upward-facing teeth 98 of control knob 72 engage the downward-facing teeth 96 of recess 74 and control knob 72 is locked in place and cannot be moved axially. As seen in Fig. 9B, when a downward force is exerted on control knob 72, the upward-facing teeth 98 of control knob 72 disengage from the downward-facing teeth 96 of recess 74 and control knob 72 is now unlocked and free to move axially.

Once the desired angle of the tubes is achieved, the user releases the downward force on control knob 72, which causes the upward-facing teeth 98 of control knob 72 to once again engage the downward-facing teeth 96 of recess 74 at the nearest engagement station, and control knob 72 is once again locked in place.

Arm 80 serves as a lever arm for linear movement amplification. For example, for each 1 mm of the external tube movement, 3 mm of movement of the control knob 72 is required. This amplified or longer travel of control knob 72 provides finer resolution of the tube deformation.

Reference is now made to Fig. 10, which illustrates a twist knob 102 of the handle 70, which is used to control the twisted shape of the internal and external tubes 12 and 14 of the steering tool by rotating the internal tube 12 about its longitudinal axis, as is now explained.

The internal tube 14 may be connected to twist knob 102 by means of an adaptor 104 (seen in Fig. 8A). Adaptor 104 may also seal the connector 73 (as seen in Fig. 8A). Rotational movement of twist knob 102 about the longitudinal axis of the handle causes rotational movement of internal tube 12 about its longitudinal axis.

Thus, control knob 72 of handle 70 serves as the tube manipulator to bend, curve or otherwise deform the distal portions of the tubes 12 and 14, and thereby control steering and tip location of the steering tool. The twist knob 102 acts as a phase shift manipulator for changing the amount of phase shift between the tubes or between the slots, and thereby cause a twisted or spiral shape to the distal tips of the tubes. The operation of control knob 72 (e.g., to cause steering and bending of the distal tips) and the operation of twist knob 102 (e.g., to cause twisting torque) are independent of each other, thereby providing the surgeon with limitless possibilities of shaping the distal tips to any desired, three-dimensional shape. The twisting torque also changes the stiffness of the bent portion of the tubes.

## Claims

1. A steering tool (10) comprising:
an internal tube (12) disposed inside an external tube (14), said internal and external tubes (12, 14) being arranged for longitudinal axial movement relative to one another, wherein a distal end of said internal tube (12) is fixedly joined to a distal end of said external tube (14);
a tube manipulator (24) operative to cause relative axial movement of said internal and external tubes (12, 14) and bending of a distal portion of at least one of said internal and external tubes (12, 14); and
a locking mechanism (18, 32, 34) operative to switch between a locked position and an unlocked position, wherein in the locked position said internal and external tubes (12, 14) are locked at a particular longitudinal axial position relative to one another, and in the unlocked position there is uninhibited free movement of said internal and external tubes (12, 14) relative to one another,
wherein said locking mechanism (18, 32, 34) comprises an adjustment assembly (38) operative to create an intermediate locked position in which there is partially inhibited movement of said internal and external tubes (12, 14) relative to one another, and
wherein said adjustment assembly (38) comprises a biasing device (38) operative to apply a biasing force on at least one of said internal and external tubes (12, 14) that at least partially inhibits movement of said internal and external tubes (12, 14) relative to one another.

2. The steering tool (10) according to claim 1, comprising a control knob (18) operatively connected to said adjustment assembly (38) for switching between said locked, intermediate locked and unlocked positions.

3. The steering tool (10) according to claim 2, wherein said control knob (18) is lockable at different positions.

4. The steering tool (10) according to claim 2, wherein said control knob (18) is connected to a stopper (32) which is movable towards an abutment element (34) mounted on at least one of said tubes (12, 14).

5. The steering tool (10) according to claim 1, wherein a control knob (18) is operatively connected to said adjustment assembly (38) for switching between said locked, intermediate locked and unlocked positions, wherein said control knob (18) is connected to a stopper (32) which is movable towards an abutment element (34) mounted on at least one of said tubes (12, 14), and wherein in the locked position, said biasing device (38) applies a first biasing force against said abutment element (34) sufficient to completely inhibit movement of said internal and external tubes (12, 14) relative to one another, and in the intermediate locked position, said biasing device (38) applies a second biasing force against said abutment element (34) to partially inhibit movement of said internal and external tubes (12, 14) relative to one another.

6. The steering tool (10) according to claim 1, wherein in said locked position, distal portions of said internal and external tubes (12, 14) are in a fixed deformed orientation, and in said intermediate locked, said distal portions of said internal and external tubes (12, 14) are in a changeable deformed orientation.

7. The steering tool (10) according to claim 1, comprising a phase shift manipulator (25) in engagement with said internal tube (12), wherein rotation of said phase shift manipulator applies torque to said internal tube (12).

8. The steering tool (10) according to claim 7, wherein said tube manipulator (24) and said phase shift manipulator (25) operate independently of each other.

9. The steering tool (10) according to claim 1, comprising a control knob (72) operatively connected to said external tube (14) by an arm (80).

10. The steering tool (10) according to claim 9, wherein said arm (80) comprises a lever arm for linear movement amplification between said control knob (72) and said external tube (14).

11. The steering tool (10) according to claim 2, wherein said locking mechanism (18, 32, 34) comprises teeth (22) formed on said control knob (18) that selectively engage with or disengage from ridges (23) formed on a portion of a handle (16).

## Patentansprüche

1. Lenkbares Werkzeug (10), umfassend:
ein inneres Rohr (12), das innerhalb eines äußeren Rohrs (14) angeordnet ist, wobei das innere und das äußere Rohr (12,14) für eine axiale Längsbewegung relativ zueinander angeordnet sind, wobei ein distales Ende des inneren Rohrs (12) fest mit einem distalen Ende des äußeren Rohrs (14) verbunden ist;
einen Rohrmanipulator (24), um betrieblich eine relative axiale Bewegung des inneren und des äußeren Rohrs (12, 14) und ein Biegen eines distalen Abschnitts von mindestens einem von dem inneren und dem äußeren Rohr (12, 14) zu bewirken; und
einen Verriegelungsmechanismus (18, 32, 34), um betrieblich zwischen einer verriegelten Position und einer entriegelten Position umzuschalten, wobei in der verriegelten Position die inneren und äußeren Rohre (12, 14) in einer bestimmten longitudinalen axialen Position relativ zueinander verriegelt sind und in der entriegelten Position eine ungehinderte freie Bewegung der inneren und äußeren Rohre (12, 14) relativ zueinander besteht,
wobei der Verriegelungsmechanismus (18, 32, 34) eine Einstellvorrichtung (38) umfasst, um betrieblich eine teil-verriegelte Position zu erzeugen, in der eine teilweise gesperrte Bewegung der inneren und äußeren Rohre (12, 14) relativ zueinander vorliegt, und
wobei die Einstellvorrichtung (38) eine Vorspannvorrichtung (38) umfasst, um betrieblich eine Vorspannkraft auf mindestens eines der inneren und äußeren Rohre (12, 14) auszuüben, die zumindest teilweise eine Bewegung der inneren und äußeren Rohre (12, 14) relativ zueinander verhindert.

2. Lenkbares Werkzeug (10) nach Anspruch 1, umfassend einen Steuerknopf (18), der betrieblich mit der Einstellvorrichtung (38) verbunden ist, um zwischen der verriegelten Position, der teil-verriegelten Position und der entriegelten Position umzuschalten.

3. Lenkbares Werkzeug (10) nach Anspruch 2, wobei der Steuerknopf (18) in verschiedenen Positionen verriegelbar ist.

4. Lenkbares Werkzeug (10) nach Anspruch 2, wobei der Steuerknopf (18) mit einem Stopper (32) verbunden ist, der in Richtung eines Anschlagelements (34) bewegbar ist, das an mindestens einem der Rohre (12, 14) angebracht ist.

5. Lenkbares Werkzeug (10) nach Anspruch 1, wobei der Steuerknopf (18) betrieblich mit der Einstellvorrichtung (38) verbunden ist, um zwischen der verriegelten Position, der teil-verriegelten Position und der entriegelten Position umzuschalten, wobei der Steuerknopf (18) mit einem Stopper (32) verbunden ist, der in Richtung eines Anschlagelements (34) bewegbar ist, das an mindestens einem der Rohre (12, 14) angebracht ist, und wobei in der verriegelten Position die Vorspannvorrichtung (38) eine erste Vorspannkraft gegen das Anschlagelement (34) ausübt, die ausreicht, um eine Bewegung der inneren und äußeren Rohre (12, 14) relativ zueinander vollständig zu verhindern, und in der teil-verriegelten Position die Vorspannvorrichtung (38) eine zweite Vorspannkraft gegen das Anschlagelement (34) ausübt, um eine Bewegung der inneren und äußeren Rohre (12, 14) relativ zueinander teilweise zu verhindern.

6. Lenkbares Werkzeug (10) nach Anspruch 1, wobei in der verriegelten Position die distalen Abschnitte der inneren und äußeren Rohre (12, 14) in einer festen verformten Orientierung sind und in der teil-verriegelten Position die distalen Abschnitte der inneren und äußeren Rohre (12, 14) in einer veränderbaren verformten Orientierung sind.

7. Lenkbares Werkzeug (10) nach Anspruch 1, mit einem Phasenschiebermanipulator (25) in Eingriff mit dem Innenrohr (12), wobei die Drehung des Phasenverschiebungsmanipulators ein Drehmoment auf das Innenrohr (12) ausübt.

8. Lenkbares Werkzeug (10) nach Anspruch 7, wobei der Rohrmanipulator (24) und der Phasenschiebermanipulator (25) unabhängig voneinander arbeiten.

9. Lenkbares Werkzeug (10) nach Anspruch 1, mit einen Steuerknopf (72), der über einen Arm (80) betreiblich mit dem außeren Rohr (14) verbunden ist.

10. Lenkbares Werkzeug (10) nach Anspruch 9, wobei der Arm (80) einen Hebelarm zur linearen Verstärkung der Bewegung zwischen dem Bedienknopf (72) und dem äußeren Rohr (14) umfasst.

11. Lenkbares Werkzeug (10) nach Anspruch 2, wobei der Verriegelungsmechanismus (18, 32, 34) Zähne (22) umfasst, die an dem Steuerknopf (18) ausgebildet sind, wobei die Zähne (22) wahlweise Rippen (23), die an einem Abschnitt eines Griffs (16) ausgebildet sind, in Eingriff nehmen oder freigeben.

## Revendications

1. Outil de guidage (10) comprenant :
un tube interne (12) disposé à l'intérieur d'un tube externe (14), lesdits tubes interne et externe (12, 14) étant agencés afin d'assurer un mouvement axial longitudinal de l'un par rapport à l'autre, dans lequel une extrémité distale dudit tube interne (12) est solidaire d'une extrémité distale dudit tube externe (14) ;
un dispositif de manipulation de tube (24) pouvant servir à produire un mouvement axial relatif desdits tubes interne et externe (12, 14) et à fléchir une partie distale d'au moins l'un desdits tubes interne et externe (12, 14) ; et
un mécanisme de verrouillage (18, 32, 34) pouvant servir à commuter entre une position verrouillée et une position déverrouillée, dans lequel, dans la position verrouillée, lesdits tubes interne et externe (12, 14) sont verrouillés à une position axiale longitudinale particulière l'un par rapport à l'autre et, dans la position déverrouillée, un mouvement libre desdits tubes interne et externe (12, 14) l'un par rapport à l'autre n'est pas entravé,
dans lequel ledit mécanisme de verrouillage (18, 32, 34) comprend un ensemble de réglage (38) pouvant servir à créer une position intermédiaire verrouillée dans laquelle un mouvement desdits tubes interne et externe (12, 14) l'un par rapport à l'autre est partiellement inhibé, et
dans lequel ledit ensemble de réglage (38) comprend un dispositif d'application (38) pouvant servir à appliquer un effort d'application sur le au moins un desdits tubes interne et externe (12, 14) qui inhibe au moins partiellement le mouvement desdits tubes interne et externe (12, 14) l'un par rapport à l'autre.

2. Outil de guidage (10) selon la revendication 1, comprenant un bouton de commande (18) relié de manière opérationnelle audit ensemble de réglage (38) afin d'assurer le passage entre lesdites positions verrouillée, intermédiaire verrouillée et déverrouillée.

3. Outil de guidage (10) selon la revendication 2, dans lequel ledit bouton de commande (18) peut être verrouillé à différentes positions.

4. Outil de guidage (10) selon la revendication 2, dans lequel ledit bouton de commande (18) est couplé à une butée (32) qui peut être déplacée vers un élément de butée (34) monté sur au moins l'un desdits tubes (12, 14).

5. Outil de guidage (10) selon la revendication 1, dans lequel un bouton de commande (18) est couplé de manière opérationnelle audit ensemble de réglage (38) afin d'assurer le passage entre lesdites positions verrouillée, intermédiaire verrouillée et déverrouillée, dans lequel ledit bouton de commande (18) est couplé à une butée (32) qui peut être déplacée vers un élément de butée (34) monté sur au moins l'un desdits tubes (12, 14), et dans lequel, dans la position verrouillée, ledit dispositif d'application (38) applique un premier effort d'application contre ledit élément de butée (34) qui est suffisant afin d'inhiber totalement le mouvement desdits tubes interne et externe (12, 14) l'un par rapport à l'autre et, dans la position intermédiaire verrouillée, ledit dispositif d'application (38) applique un second effort d'application contre ledit élément de butée (34) afin d'inhiber partiellement le mouvement desdits tubes interne et externe (12, 14) l'un par rapport à l'autre.

6. Outil de guidage (10) selon la revendication 1, dans lequel, dans ladite position verrouillée, des parties distales desdits tubes interne et externe (12, 14) sont suivant une orientation déformée fixe et, dans ladite position intermédiaire verrouillée, lesdites parties distales desdits tubes interne et externe (12, 14) sont suivant une orientation déformée pouvant être modifiée.

7. Outil de guidage (10) selon la revendication 1, comprenant un dispositif de manipulation à déphasage (25) couplé avec ledit tube interne (12), dans lequel la rotation dudit dispositif de manipulation à déphasage applique un couple sur ledit tube interne (12).

8. Outil de guidage (10) selon la revendication 7, dans lequel ledit dispositif de manipulation de tube (24) et ledit dispositif de manipulation à déphasage (25) fonctionnent de manière indépendante l'un par rapport à l'autre.

9. Outil de guidage (10) selon la revendication 1, comprenant un bouton de commande (72) couplé de manière opérationnelle audit tube externe (14) par un bras (80).

10. Outil de guidage (10) selon la revendication 9, ledit bras (80) comprend un bras de levier afin d'assurer une amplification de mouvement linéaire entre ledit bouton de commande (72) et ledit tube externe (14).

11. Outil de guidage (10) selon la revendication 2, dans lequel ledit mécanisme de verrouillage (18, 32, 34) comprend des dents (22) formées sur ledit bouton de commande (18) qui se couplent ou se séparent de manière sélective de nervures (23) formées sur une partie d'une poignée (16).
